# EUROPEAN PATENT APPLICATION

(11) **EP 4 101 924 A1**
(43) Date of publication of application: **14.12.2022**
(21) Application number: 21750181.6
(22) Date of filing: 05.02.2021
(51) Int. Cl.: C12N 5/0783, C12N 5/10, C12N 15/09

(54) **CYTOTOXIC T CELLS DERIVED FROM HUMAN T CELL-DERIVED IPS CELLS**

(30) Priority: 07.02.2020 JP 2020019548
(71) Applicant: Juntendo Educational Foundation, Tokyo 113-8421 (JP); The University of Tokyo, Bunkyo-ku, Tokyo 113-8654 (JP)
(72) Inventor: ANDO, Miki, Tokyo 113-8421 (JP); ANDO, Jun, Tokyo 113-8421 (JP); ISHII, Midori, Tokyo 113-8421 (JP); KOMATSU, Norio, Tokyo 113-8421 (JP); NAKAUCHI, Hiromitsu, Tokyo 113-8654 (JP); WATANABE, Motoo, Tokyo 113-8654 (JP)
(74) Representative: Plougmann Vingtoft a/s
(86) International application number: PCT/JP2021/004232
(87) International publication number: WO 2021/157685

(57) **Abstract**

Provided are cytotoxic T cells derived from human T cell-derived iPS cells that can avoid an NK cell missing-self response and that can be used for allogeneic administration while maintaining a strong antitumor effect of antigen-specific CTLs, and a method for producing the same.

A method for producing a cytotoxic T cell derived from a human T cell-derived iPS cell expressing HLA class I of HLA-restricted class I of an antigen epitope of a CTL and HLA-E, the method including: a step of knocking out all HLA class I of the human T cell-derived iPS cell; a step of introducing a gene of the HLA-restricted HLA class I of the antigen epitope of the CTL and a gene of the HLA-E into the T-iPS cell in which all HLA class I have been knocked out; and a step of redifferentiating the genetically introduced T-iPS cell into a CD8 single-positive T cell.

## Description

### Technical Field

The present invention relates to cytotoxic T cells derived from human T cell-derived iPS cells that can be used for allogeneic administration and a method for producing the same.

### Background Art

Antigen-specific cytotoxic T cells (CTLs) recognize an antigen peptide derived from, for example, a virus or a tumor presented together with major histocompatibility class 1 antigens (MHC class I, HLA class I) of antigen-presenting cells via a T cell receptor (TCR) present on the cell surface of the CTLs, specifically exerts cytotoxic activity against and attacks cells presenting the antigen peptide as a foreign substance. Some of the CTLs become long-lived memory T cells, are stored in the host while maintaining cytotoxicity against foreign substances, and then can respond to exposure to foreign substances. Therefore, CTLs are expected as cells for immune cell therapy in patients with viral infection and cancer diseases.

In patients with chronic virus infection and cancer patients, T cells are exhausted and aged due to chronic exposure to antigens, and cannot exert the effect, and thus the effect of T cell therapy is not obtained in many cases. Thus, iPS cell-derived rejuvenated T cell therapy in which exhausted T cells are functionally rejuvenated using the iPS technology and the obtained rejuvenated T cells are administered to a patient is expected to be an effective means for improving the cancer therapeutic effect. As a means for obtaining CTLs for use in this rejuvenated T cell therapy, a method for establishing T cell-derived iPS cells (T-iPS cells) from T cells having antigen specificity and inducing differentiation into, for example, CTLs again and chimeric antigen receptor T cells (CARTs) while maintaining the recombinant structure of the TCR gene of the original T cells has been developed (Patent Literature 1).

However, these methods have problems in which it takes 5 months to prepare rejuvenated T cells and the preparation cost is also high. On the other hand, if iPS cells derived from allogeneic CTLs are stocked in advance and rejuvenated T cells (rejT) are prepared, the rejT can be more quickly administered to a patient, and the cost per patient can be reduced. However, if HLA is not matched, the rejT is rejected, posing a problem of attenuation of an antitumor effect.

In order to solve this problem, if allogeneic rejT in which HLA class I expression is eliminated and which are not rejected by patient CD8+ T cells are prepared, it is possible to rapidly administer the allogeneic rejT to many severe patients while maintaining a strong antitumor effect of antigen-specific CTLs. Regarding allogeneic cells derived from healthy donors, in order to be able to administer iPS cells that have been validated and stocked to many patients, a method for knocking out B2M by HLA genome editing to eliminate HLA class I antigens is promising; however, in this case, it is necessary to suppress an NK cell missing-self response. As this means, several editing methods for avoiding a missing-self response of NK cells have already been reported, such as a means for eliminating HLA class I of iPS cells to induce differentiation of cells expressing only HLA-E, thereby avoiding attack from NK cells (Non Patent Literature 1), a means for eliminating HLA class I and class II and expressing PD-L1, HLA-G, and "don't eat me" signal CD47 (Non Patent Literature 2), and a means for eliminating HLA-A and -B and retaining HLA-C (Non Patent Literature 3).

### Citation List

### Patent Literature

Patent Literature 1: JP 6164746 B2

### Non Patent Literature

Non Patent Literature 1: Gornalusse GG, Hirata RK, Funk SE, Riolobos L, Lopes VS, Manske G, et al. HLA-E-expressing pluripotent stem cells escape allogeneic responses and lysis by NK cells. Nature biotechnology. 2017;35(8):765-72. Non Patent Literature 2: Han X, Wang M, Duan S, Franco PJ, Kenty JH, Hedrick P, et al. Generation of hypoimmunogenic human pluripotent stem cells. Proceedings of the National Academy of Sciences of the United States of America. 2019;116(21):10441-6.
Non Patent Literature 3: Xu H, Wang B, Ono M, Kagita A, Fujii K, Sasakawa N, et al. Targeted Disruption of HLA Genes via CRISPR-Cas9 Generates iPSCs with Enhanced Immune Compatibility. Cell stem cell. 2019;24(4):566-78 e7.

### Summary of Invention

### Technical Problem

However, any means was not sufficiently satisfactory in terms of the balance between avoidance of an NK cell missing-self response and retention of original antigen-specific CTL activity.

Therefore, an object of the present invention is to provide cytotoxic T cells derived from human T cell-derived iPS cells that can avoid an NK cell missing-self response and that can be used for allogeneic administration while maintaining a strong antitumor effect of antigen-specific CTLs, and a method for producing the same.

### Solution to Problem

Therefore, the present inventors have found that the activity of NK cells can be more strongly suppressed by expressing an HLA-restricted class I molecule (for example, HLA-A24 for an HLA-A24-restricted CTL, and HLA-A02 for an HLA-A02-restricted CTL) of an antigen epitope of a CTL and HLA-E in human T cell-derived iPS cells, and have completed the present invention.

That is, the present invention provides the following [1] to [4].
[1] A cytotoxic T cell derived from a human T cell-derived iPS cell expressing an HLA-restricted class I molecule of an antigen epitope of a CTL and HLA class I of HLA-E.
[2] The cytotoxic T cell derived from a human T cell-derived iPS cell according to [1], wherein the HLA-restricted class I molecule of the antigen epitope of the CTL is HLA-A24 or HLA-A02.
[3] A method for producing a cytotoxic T cell derived from a human T cell-derived iPS cell expressing two types of HLA class I of an HLA-restricted class I molecule of an antigen epitope of a CTL and HLA-E, the method including: a step of knocking out all HLA class I of the human T cell-derived iPS cell; a step of introducing a gene of the HLA-restricted class I molecule of the antigen epitope of the CTL and a gene of the HLA-E into the T cell in which all HLA class I have been knocked out; and a step of redifferentiating the genetically introduced T-iPS cell into a CD8 single-positive T cell.
[4] The production method according to [3], wherein the HLA-restricted class I molecule of the antigen epitope of the CTL is HLA-A24 or HLA-A02.

### Advantageous Effects of Invention

According to the present invention, it is possible to stably provide cytotoxic T cells derived from human T cell-derived iPS cells that can avoid an NK cell missing-self response and that can be used for allogeneic administration while maintaining a strong antitumor effect of antigen-specific CTLs, and a method for producing the same.

### Brief Description of Drawings

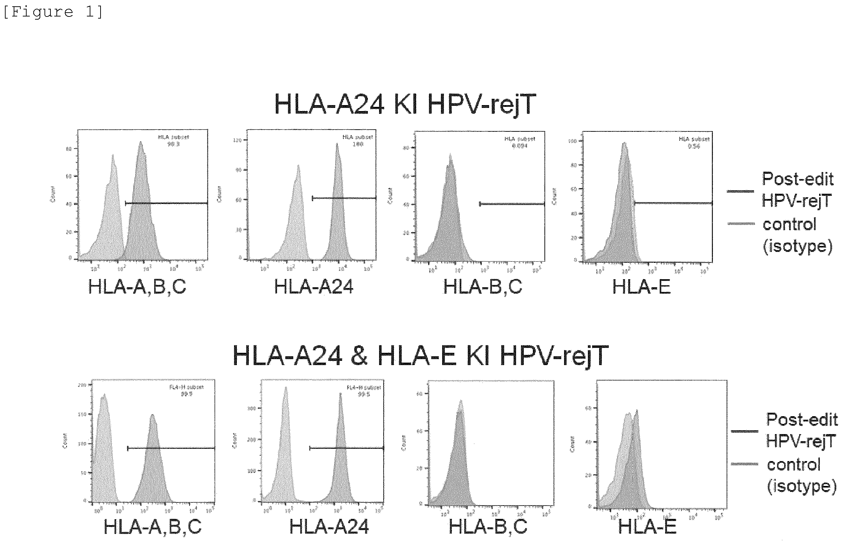

Fig. 1 is a diagram showing HLA class I expression of HPV-rejT after genome editing. Post-edit HPV-rejT represents HPV-rejT in which only HLA-A24 was knocked in after elimination of HLA class I (top) and HPV-rejT expressing both HLA-A24 and HLA-E (bottom). HPV-rejT was stained using antibodies against respective HLA class 1 (ABC, A24, BC, and E) and analyzed by flow cytometry. Control (isotype) represents a negative control of HPV-rejT stained with an isotype control.

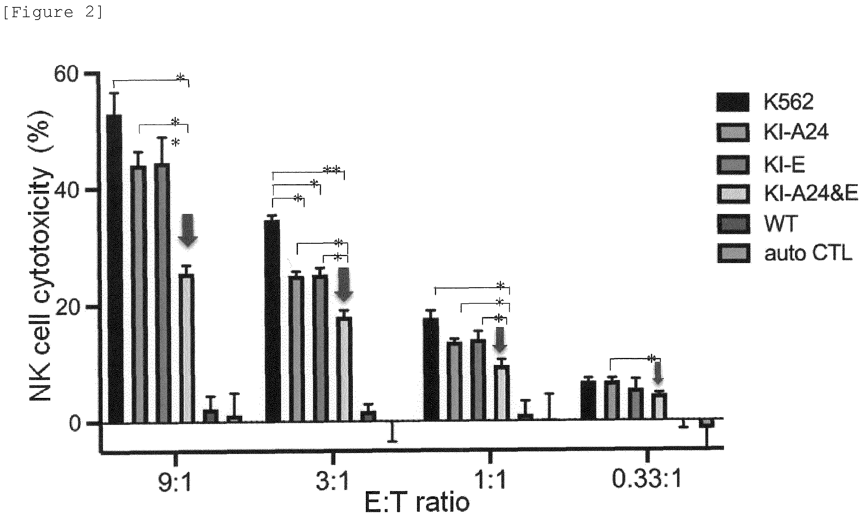

Fig. 2 is a diagram showing that HPV-rejT in which both HLA-A24 and HLA-E are expressed significantly suppresses the cytotoxic activity of NK cells. Since a K562 cell line does not express class I, the K562 cell line is a positive control that is injured by NK cells. KI-A24 represents HPV-rejT in which only HLA-A24 was knocked in after elimination of HLA class I. KI-E represents HPV-rejT in which only HLA-E was knocked in after elimination of HLA class I. KI-A24&E represents HPV-rejT in which both HLA-A24 and HLA-E were knocked in after elimination of HLA class I. WT represents a wild type in which HLA is not edited, that is, which expresses HLA. Auto CTL represents CTLs derived from a donor of NK cells used for this analysis. Since the auto CTLs are autologous cells, NK cells do not attack the auto CTLs, and therefore the auto CTLs are used as a negative control.

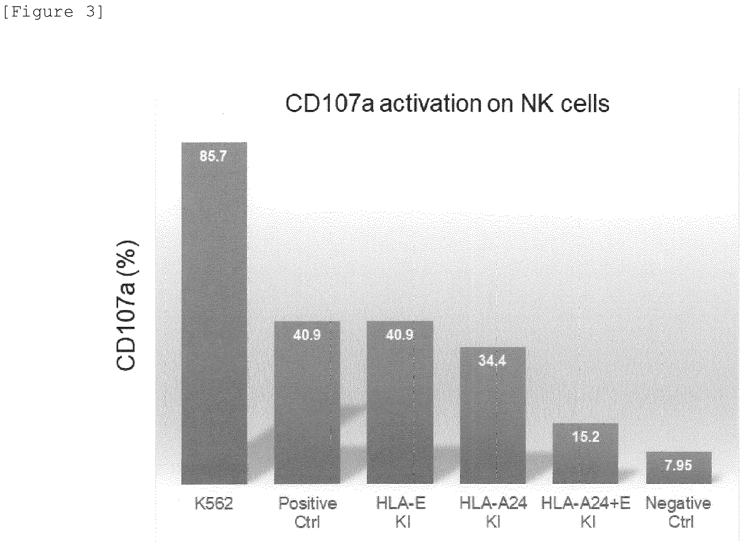

Fig. 3 is a diagram showing that the inhibitory effect on the NK cell cytotoxic activity of HLA-A24+HLA-E-expressing HPV-rejT is strong also in a 107a assay. Since the K562 cell line does not express class I, NK cells attack K562 by co-culture to highly express 107a. The K562 cell line is used as a positive control. Regarding Positive Ctrl, since NK cells were stimulated by adding Cell Stimulation Cocktail, the NK cells highly express 107a. The Positive Ctrl also represents a positive control. HLA-E KI represents HPV-rejT in which only HLA-E was knocked in after elimination of HLA class I. HLA-A24 KI represents HPV-rejT in which only HLA-A24 was knocked in after elimination of HLA class I. HL-A24+E KI represents HPV-rejT in which both HLA-A24 and HLA-E were knocked in after elimination of HLA class I. Negative Ctrl represents a negative control cultured with only NK cells.

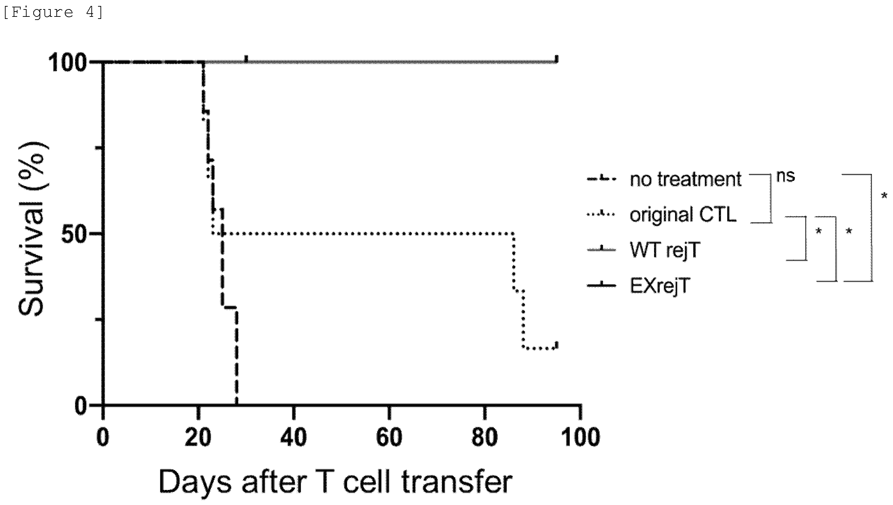

Fig. 4 shows an effect of prolonging the survival time of HLA-edited HPV-rejT in cervical cancer-bearing mice. No treatment represents a non-treated group, original CTL represents an original CTL-treated group, WTrejT represents a rejT-treated group, and EXrejT represents a group treated with the HPV-rejT of the present invention.

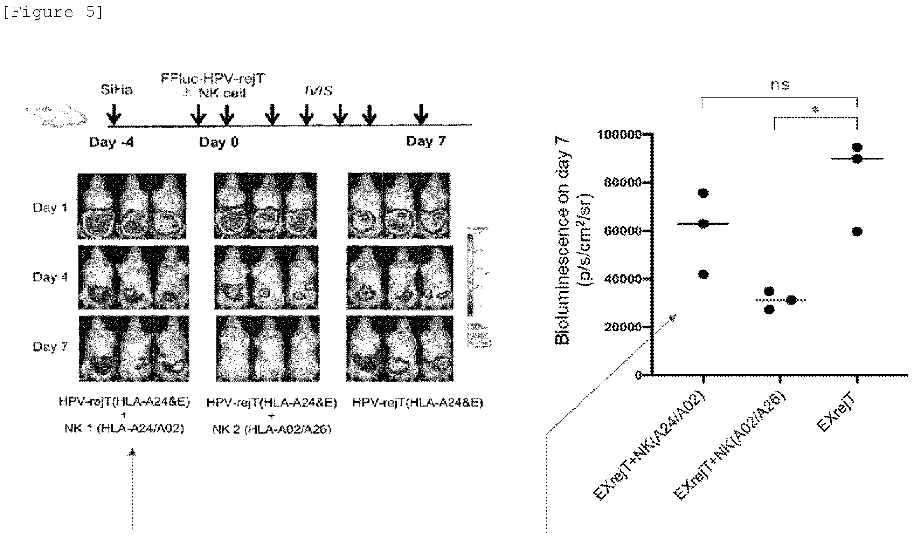

Fig. 5 shows durability in vivo of HLA-edited HPV-rejT upon co-administration of NK cells. Characters in parentheses in HPV-rejT represent HLA class I editing.

### Description of Embodiments

Cytotoxic T cells of the present invention are cytotoxic T cells in which HLA of a human T cell-derived iPS cell (T-iPS cell) is modified to express an HLA-restricted class I molecule of an antigen epitope of a CTL and HLA class I of HLA-E.

In addition to the HLA-restricted class I molecule of the antigen epitope of the CTL and HLA-E, the cytotoxic T cells of the present invention may further express HLA class I such as HLA-G and HLA-C, and may further express, for example, CD47, PD-L1, and iCaspase9. However, from the viewpoint of suppression of NK cell activity and from the viewpoint of HLA matching with a donor due to HLA polymorphism, preferred are cytotoxic T cells derived from human T cell-derived iPS cells expressing two types of HLA class I of the HLA-restricted class I molecule of the antigen epitope of the CTL and HLA-E.

The human T cell-derived iPS cells used as a raw material can be obtained by inducing human T cells into iPS cells (T-iPS cells). This method for producing T-iPS cells is preferably performed by the method described in Patent Literature 1.

First, means for inducing human T cells into iPS cells will be described.

T cells used are preferably human T cells. A human that is the origin of the T cells may be a human suffering from, for example, viral infection or malignant tumor; however, the human is preferably a healthy person from the viewpoint that the cells are banked after genome editing and administered to many people in the production of therapeutic alloantigen-specific cytotoxic T cells. The type of HLA in a preferable human as the origin of T cells does not need to completely match that of a patient who should receive regenerated CTLs or CART cells produced using T-iPS cells that have been produced according to the present invention.

T cells to be induced into T-iPS cells in the present invention are preferably T cells having antigen specificity. Examples of the T cells include T cells expressing CD3 and CD8, and specifically CTLs that are CD8-positive cells. Examples of the T cells include T cells expressing CD3 and CD4, and specifically T cells that are CD4-positive cells. The antigen specificity in T cells is provided by an antigen-specific, rearranged TCR gene. From the viewpoint of production efficiency, although not particularly limited, it is preferable to use antigen-specific CD8-positive T cells as human T cells to be induced into T-iPS cells in order to obtain antigen-specific CD8-positive cells, and it is preferable to use antigen-specific CD4-positive T cells as human T cells to be induced into T-iPS cells in order to obtain antigen-specific CD4-positive cells. When immunotherapy is performed, it is preferable that human T cells to be differentiated from iPS cells have the same or substantially the same antigen specificity as that of human T cells to be induced into iPS cells. T cells having no antigen specificity are also included as T cells that induce T-iPS cells. Specific examples of the T cells include genetically modified T cells such as CART cells or TCR-T cells.

Such T cells can be isolated, for example, from human tissues by a known method.

Examples of the human tissues include tissues containing the T cells, for example, peripheral blood, lymph nodes, bone marrow, thymus, spleen, umbilical cord blood, and lesion tissues. Among them, peripheral blood is preferable from the viewpoint of low invasiveness to humans and ease of preparation. When tumor infiltrating lymphocytes (TILs) are separated, they can be separated from tumor tissues or peripheral blood. Examples of known methods for isolating human T cells include magnetic selection using, for example, magnetic beads for cell separation, flow cytometry using an antibody against a cell surface marker such as CD4 or CD8 and a cell sorter, and an activated T cell induction method using an anti-CD3 antibody or an anti-CD28 antibody. It is also possible to isolate desired T cells by using cytokine secretion, functional molecule expression, or a signal molecule such as PD-1 as an index. It is possible to isolate cytotoxic T cells (CTLs) by using secretion or production of, for example, granzyme or perforin as an index. Furthermore, in the case of isolation from human tissues containing T cells having antigen specificity, T cells having desired antigen specificity can be purified from human tissues using a multimerized product of major histocompatibility complex (MHC) to which a desired antigen is bound (for example, "MHC tetramer", "Pro5 (registered trademark) MHC class I pentamer").

In the present invention, a gene to be introduced to convert T cells into iPS cells is preferably a combination of at least four types of genes among genes such as (a) Oct3/4 gene, (b) c-Myc gene, (c) Sox2 gene, (d) Klf4 gene, (e) NANOG gene, and (f) LIN28 gene.

In the present invention, a method for introducing the gene group into T cells is not particularly limited, and a known method can be appropriately selected and used. For example, when the gene group in a form of a nucleic acid encoding the gene group is introduced into the T cells, a nucleic acid (for example, cDNA, RNA) encoding the gene group can be inserted into an appropriate expression vector containing a promoter that functions in T cells, and the expression vector can be introduced into cells by infection, a lipofection method, a liposome method, an electroporation method, a calcium phosphate co-precipitation method, a DEAE dextran method, a microinjection method, or an electroporation method.

Among such expression vectors, it is more preferable to use a stealth RNA expression vector containing the gene group from the viewpoint of reduction of a risk of canceration and introduction efficiency.

The stealth RNA expression vector is a vector designed to avoid entry of the vector into a chromosome and to express a gene continuously and stably in cytoplasm rather than in nucleus. It is possible to introduce a large gene of 13,000 base pairs or more or simultaneously introduce 10 genes; the vector does not damage cells; an introduced gene can be removed when unnecessary; and the vector has a stealth property in which the cell cannot recognize the vector as a foreign substance.

Examples of such a stealth RNA expression vector include a complex that is composed of a negative-sense single-stranded RNA (A) containing RNA sequences of the following (1) to (8), a single-stranded RNA-binding protein (B), and an RNA-dependent RNA synthetase and that does not activate an innate immune structure.
(1) an RNA sequence for the gene group,
(2) a human mRNA-derived RNA sequence constituting a non-coding region,
(3) a transcription initiation signal sequence recognized by the RNA-dependent RNA synthetase,
(4) a transcription termination signal sequence recognized by the RNA-dependent RNA synthetase,
(5) an RNA sequence containing a replication origin recognized by the RNA-dependent RNA synthetase,
(6) an RNA sequence encoding the RNA-dependent RNA synthetase,
(7) an RNA sequence encoding a protein that regulates the activity of the RNA-dependent RNA synthetase,
   and
(8) an RNA sequence encoding the single-stranded RNA-binding protein.

When T-iPS cells are established, the T cells are preferably stimulated and activated by an anti-CD3 antibody and an anti-CD28 antibody in the presence of interleukin-2 (IL-2) or interleukin-7 (IL-7) and interleukin-15 (IL-15) before introduction of the gene group, and may be stimulated and activated by at least one substance selected from the group consisting of phytohemagglutinin (PHA), interleukin-2 (IL-2), an alloantigen-expressing cell, an anti-CD3 antibody, an anti-CD28 antibody, and CD3 and CD28 agonists. Such stimulation can be performed, for example, by adding, for example, PHA, IL-2, an anti-CD3 antibody, and/or an anti-CD28 antibody to a medium and culturing the T cells for a certain period of time. The anti-CD3 antibody and the anti-CD28 antibody may be those to which, for example, magnetic beads are bound, and stimulation may be given by culturing the T cells for a certain period of time on a culture dish, to the surface of which the anti-CD3 antibody and the anti-CD28 antibody are bound, instead of adding these antibodies to the medium. Furthermore, stimulation may be given by adding an antigen peptide recognized by the T cells (for example, human T cells) to the medium together with feeder cells.

In order to give such stimulation to the T cells, the concentration of PHA added to the medium is not particularly limited; however, the concentration is preferably from 1 to 100 µg/mL. The concentration of IL-2 added to the medium is not particularly limited; however, the concentration is preferably from 1 to 200 ng/mL.

Furthermore, the concentrations of the anti-CD3 antibody and the anti-CD28 antibody added to the medium are not particularly limited; however, the concentrations are preferably from 1 to 10 times the culture amount of the T cells. In order to give such stimulation to the T cells, the concentrations of the anti-CD3 antibody and the anti-CD28 antibody bound to the surface of the culture dish are not particularly limited; however, it is preferable that the concentration at the time of coating is from 0.1 to 100 µg/mL, and preferably from 1 to 100 µg/mL for the anti-CD3 antibody, and from 0.1 to 10 µg/mL for the anti-CD28 antibody.

The culture period for giving such stimulation is not particularly limited as long as it is a period sufficient for giving such stimulation to the T cells and is a period during which the T cells can be expanded to the number of cells necessary for introduction of the four genes; however, the culture period is usually from 2 to 7 days, and is preferably from 3 to 5 days from the viewpoint of gene introduction efficiency. From the viewpoint of infection by mixing T cells and a vector in a 15-mL tube or increasing gene introduction efficiency, it is preferable to culture on a culture dish coated with RetroNectin.

As the medium in which the T cells are cultured and to which for example, PHA, IL-2, the anti-CD3 antibody, and/or the anti-CD28 antibody are added, for example, a known medium suitable for culturing the T cells, more specifically, a Roswell Park Memorial Institute (RPMI) 1640 medium containing other cytokines and human serum, an AIM V^{™} medium, or NS-A2 can be used. In addition to PHA, IL-2, the anti-CD3 antibody, and/or the anti-CD28 antibody, an amino acid (for example, L-glutamine) and an antibiotic (for example, streptomycin, penicillin) required for culture may be added to the medium. It is also preferable to add IL-7 and IL-15 to the medium instead of IL-2. The concentration of each of IL-7 and IL-15 added is not particularly limited; however, the concentration is preferably from 1 to 100 ng/mL.

Conditions at the time of introducing the four genes into the T cells or conditions after the introduction are not particularly limited; however, the T cells into which the four genes have been introduced are preferably cultured under feeder-free conditions. Examples of the conditions include a well coated with iMatrix-511 solution, which is a Laminin-511E8 fragment, or vitronectin. Establishment is possible even by culture under feeder cell conditions, and examples of the feeder cells include mouse embryonic fibroblasts (MEFs), STO cells, and SNL cells whose cell division has been stopped by irradiation with radiation or antibiotic treatment.

Furthermore, in the process of inducing T-iPS cells from the T cells, it is preferable to add a medium for iPS cells from the next day. Thereafter, it is preferable to replace the medium by half every other day and gradually replace the T cell medium with the iPS medium.

It is preferable to culture while gradually replacing a known medium suitable for culturing the T cells with a medium suitable for culturing iPS cells in accordance with the transition from the T cells to iPS cells. As such a medium suitable for culturing iPS cells, a known medium can be appropriately selected and used, and for example, StemFit AK03N when the well is coated with iMatrix, Essential 8 Medium when the well is coated with vitronectin, or a Dulbecco's modified Eagle's medium/F12 medium (human iPS cell medium) containing, for example, a knockout serum alternative, L-glutamine, non-essential amino acids, 2-mercaptoethanol, and b-FGF for culture on feeder cells such as MEF cells is desirable.

In this way, selection of T-iPS cells can be performed by appropriately selecting a known method. Examples of such a known method include a method for selecting by observing the morphology of ES cell/iPS cell-like colonies under a microscope. Meanwhile, in the case of T-iPS established from a CTL clone that is a single cell, since the properties are often similar, there is also a method in which each colony of T-iPS cells is not selected and all established colonies are passaged as they are.

Confirmation that the cells thus selected are T-iPS cells can be performed by, for example, a method for detecting the expression of an undifferentiated cell-specific marker (for example, ALP, SSEA-4, Tra-1-60, and Tra-1-81) in the selected cells by, for example, immunostaining or RT-PCR, or a method for transplanting the selected cells into a mouse and observing the teratoma formation in the mouse. Confirmation that the cells thus selected are derived from the T cells can be performed by detecting the state of TCR gene rearrangement by genomic PCR.

Regarding a period during which these cells are selected and collected, it is preferable to collect the cells while observing the growth state of the colony. Generally, the period is from 10 to 40 days, and preferably from 14 to 28 days after the gene group containing the four genes is introduced into the T cells. A culture environment is preferably a condition of 5% CO₂ and from 35 to 38°C, and more preferably a condition of 5% CO₂ and 37°C unless otherwise specified.

In order to obtain T cells expressing a gene of HLA-restricted class I of an antigen epitope of a CTL and HLA class I of HLA-E from the human T cell-derived iPS cells obtained as mentioned above, for example, there is a method including: (a) a step of knocking out all HLA class I of the human T cell-derived iPS cells, and (b) a step of introducing the gene of HLA-restricted class I of the antigen epitope of the CTL and a gene of HLA-E into the T-iPS cells in which all HLA class I have been knocked out.

The knockout step (a) and the gene introduction step (b) can be performed by various methods, and a CRISPR-Cas9 genome editing method is one option.

First, in order to knock out all HLA class I of the human T cell-derived iPS cells by the CRISPR-Cas9 genome editing method, β2 microglobulin (B2M) is first knocked out. Five micrograms each of a knockout plasmid and a guide RNA are electroporated into about 2 × 10⁵ T-iPSCs after cell detachment. Thereafter, the cells are seeded in 3 wells of a 6-well plate and cultured. The knockout plasmid contains a target sequence of the guide RNA used for the second editing, GFP, and selection markers such as CD8 and CD19. After about 10 days, positive selection of the selection markers is performed at a timing when the cells seeded in 3 wells become confluent. After selection, iPS cells are single-cell cloned by thinly seeding the T-iPSCs. GFP strongly positive cells are picked up and then cultured, and genotyping is performed. A clone having a marker biallelically is identified by PCR and then expanded, and the process proceeds to the next step.

Next, in the step (b) of introducing the gene of HLA-restricted class 1 and the gene of HLA-E into the T-iPS cell in which all HLA class I have been knocked out by the CRISPR-Cas9 genome editing method, 2.5 µg each of two types of knock-in plasmids and 5 µg of a guide RNA are electroporated after cell detachment of the T-iPSC in which B2M has been knocked out in the first editing. After electroporation, the cells are seeded in 3 wells of a 6-well plate and cultured. After about 7 days, negative selection of the selection markers is performed at a timing when the cells seeded in 3 wells become confluent. After selection, iPS cells are single-cell cloned by thinly seeding the T-iPS cells. GFP-negative cells are picked up and then cultured, and genotyping is performed. A clone having a marker biallelically is identified by PCR and then expanded. In addition to the HLA-restricted class I molecule of the antigen epitope of the CTL and HLA-E, HLA class I such as HLA-G and HLA-C may be expressed, and, for example, CD47, PD-L1, and iCaspase9 may be further expressed by the same means as mentioned above.

Next, CTL cells are induced to differentiate from the T-iPS cells after genome editing.

As the method for inducing redifferentiation, a method for differentiating T-iPS cells into CD8+ single-positive T cells is preferable, and a method for differentiating T-iPS cells into CD4/CD8 double-negative T cells and then differentiating the CD4/CD8 double-negative T cells into CD8+ single-positive T cells is more preferable.

Furthermore, as described in Patent Literature 1, it is preferable to differentiate T-iPS cells into CD4/CD8 double-negative cells, add a substance that stimulates a T cell receptor to stimulate the CD4/CD8 double-negative cells, and then differentiate the CD4/CD8 double-negative cells that have stimulated the T cell receptor into CD8 single-positive T cells in the presence of cytokines of IL-7 and IL-15, to thereby obtain the CD8 single positive T cells.

In order to differentiate T-iPS cells into CD4/CD8 double-negative cells, it is preferable to culture T-iPS cells on feeder cells (preferably mouse stromal cells) in a medium containing, for example, cytokines, serum (for example, fetal bovine serum (FBS)), insulin, transferrin, sodium selenite, L-glutamine, α-monothioglycerol, and ascorbic acid.

The stromal cells used are preferably OP9 cells and 10T1/2 cells (C3H10T1/2 cells) that have been subjected to treatment such as irradiation. The cytokine added to the medium is preferably at least one cytokine selected from the group of VEGF, SCF, TPO, SCF, and FLT3L, and more preferably VEGF, SCF, and TPO, or VEGF, SCF, and FLT3L. Examples of the medium include an X-VIVO medium, an Iscove's modified Dulbecco's medium (IMDM medium), α-MEM, and DMEM, and the IMDM medium is preferable from the viewpoint of making it easy to form a T-iPS sack (bag-like structure containing hematopoietic progenitor cells). A culture period of the T-iPS cells is preferably from 8 to 14 days, and more preferably from 10 to 14 days after the initiation of the culture of the T-iPS cells. A culture environment is not particularly limited; however, the culture environment is preferably a condition of 5% CO₂ and from 35 to 38°C, and more preferably a condition of 5% CO₂ and 37°C. It is more preferable to culture for about 1 week under low oxygen concentration conditions (oxygen concentration: for example, from 5 to 20%).

In order to differentiate T-iPS cells into CD4/CD8 double-negative cells, it is preferable to further culture cells contained in the T-iPS sack on feeder cells (preferably stromal cells, more preferably human stromal cells) in a medium containing, for example, cytokines and serum (for example, FBS). Cells present inside the T-iPS sack can be separated, for example, by passing through a sterilized sieve instrument (for example, cell strainer). The stromal cells used for this culture are preferably OP9-DL1 cells, OP9-DL4 cells, 10T1/2/DL4 cells, and 10T1/2/DL1 cells that have been subjected to treatment such as irradiation from the viewpoint of inducing differentiation into T lymphocytes via notch signals. Examples of the cytokine added to the medium include IL-7, FLT3L, VEGF, SCF, TPO, IL-2, and IL-15. Examples of the medium include an α-MEM medium, a DMEM medium, and an IMDM medium, and an α-MEM medium is preferable. In addition to IL-7 and FLT3L, an amino acid (for example, L-glutamine) and an antibiotic (for example, streptomycin, penicillin) required for culture may be added to the medium.

A culture period of the cells contained in the T-iPS sack is preferably a period until a T cell receptor (TCR) is expressed on the cell surface of the CD4/CD8 double-negative cells obtained by differentiating in this manner, and is preferably from 14 to 28 days from the initiation of the culture of the cells contained in the T-iPS sack. A culture environment is not particularly limited; however, the culture environment is preferably a condition of 5% CO₂ and from 35 to 38°C, and more preferably a condition of 5% CO₂ and 37°C.

Whether or not a T cell receptor (TCR) is expressed on the cell surface of the CD4/CD8 double-negative cells can be evaluated by flow cytometry using an anti-TCRαβ antibody, an anti-CD3 antibody, an anti-CD4 antibody, and an anti-CD8 antibody.

In a method for producing human CD8 single-positive cells having antigen specificity, by stimulating CD4/CD8 double-negative cells derived from T-iPS cells via the TCR expressed on the cell surface, further rearrangement of a TCRA gene can be suppressed, and furthermore, in the CD8 single-positive cells obtained by redifferentiation, the frequency of appearance of T cells having the same rearrangement pattern of a TCR gene as that of the original human T cells can be extremely increased.

As a method for stimulating a T cell receptor of CD4/CD8 double-negative cells derived from T-iPS cells, preferred is a method for contacting at least one substance selected from the group consisting of an anti-CD3 antibody, an anti-CD28 antibody, an antigen peptide to which human T cells that are the origin of T-iPS cells specifically binds, cells expressing a complex with HLA exhibiting restriction to the T cell receptor, and an MHC multimer to which the antigen peptide is bound with CD4/CD8 double-negative cells derived from T-iPS cells, and more preferred is a method for contacting a specific peptide/HLA complex-expressing cell from the viewpoint of giving physiological stimulation. From the viewpoint of emphasizing uniformity of stimulation, a method for contacting an antibody or a reagent is more preferable.

The contacting method can be performed, for example, by adding, for example, PHA to a medium and culturing the T cells for a certain period of time. The anti-CD3 antibody and the anti-CD28 antibody may be those to which, for example, magnetic beads are bound, and stimulation may be given by culturing the T cells for a certain period of time on a culture dish, in the surface of which the anti-CD3 antibody and the anti-CD28 antibody are bound, instead of adding these antibodies to the medium. Furthermore, stimulation may be given by adding the antigen peptide to the medium together with feeder cells.

In order to stimulate the TCR of CD4/CD8 double-negative cells, the concentration of PHA added to the medium is preferably from 1 to 100 µg/ml. The concentrations of the anti-CD3 antibody and the anti-CD28 antibody added to the medium are preferably from 1 to 10 times the culture amount of the T cells. In order to stimulate the TCR of the CD4/CD8 double-negative cells, as the concentrations of the anti-CD3 antibody and the anti-CD28 antibody bound to the surface of the culture dish, the concentrations at the time of coating are preferably from 0.1 to 100 µg/ml for the anti-CD3 antibody and from 0.1 to 10 µg/ml for the anti-CD28 antibody.

The culture period of the cells contained in the T-iPS sack preferably includes a period necessary for expression of a T cell receptor (TCR) on the cell surface of the CD4/CD8 double-negative cells obtained by differentiating in this manner, and is preferably from 7 to 29 days from the initiation of the culture of the cells contained in the T-iPS sack. The culture environment is preferably a condition of 5% CO₂ and from 35 to 38°C, and more preferably a condition of 5% CO₂ and 37°C.

In the present invention, in order to differentiate the CD4/CD8 double-negative cells that have stimulated a T cell receptor into CD8 single-positive cells, the CD4/CD8 double-negative cells are preferably cultured in a medium containing, for example, cytokines and serum (for example, human serum). The cytokine added to the medium may be any cytokine that can differentiate CD4/CD8 double-negative cells into CD8 single-positive cells, and examples thereof include IL-7 and IL-15. Among them, IL-7 and IL-15 are preferably added in combination from the viewpoint of allowing a CD8 lineage to be selected and facilitating generation of memory-type CD8+ T cells in differentiation into CD8 single-positive cells. The concentrations of IL-7 and IL-15 added are preferably from 1 to 20 ng/ml. Examples of the medium include an RPMI-1640 medium, an X-VIVO medium, a DMEM medium, and an α-MEM medium, and an RPMI-1640 medium or an X-VIVO medium is preferable. In addition to IL-7, IL-15, and the like, an amino acid (for example, L-glutamine), an antibiotic (for example, streptomycin, penicillin), a cytokine other than IL -7, IL -15, and the like required for culture may be added to the medium.

In such culture, the CD4/CD8 double-negative cells may be co-cultured with feeder cells. The feeder cells are preferably peripheral blood mononuclear cells (PBMCs). Such PBMCs are preferably in an allogeneic relationship with the CD4/CD8 double-negative cells. From the viewpoint of continuing to stimulate the TCR and continuing to suppress further rearrangement of the TCR, it is more preferable to use peripheral blood mononuclear cells that present an antigen peptide to which human T cells that are the origin of the CD4/CD8 double-negative cells specifically bind.

A culture period for differentiating the CD4/CD8 double-negative cells into CD8 single-positive cells is preferably from 2 to 4 weeks. A culture environment is preferably a condition of 5% CO₂ and from 35 to 38°C, and more preferably a condition of 5% CO₂ and 37°C.

Confirmation that the CD8 single-positive cells thus induced to differentiate are derived from T-iPS cells and derived from T cells that are the origin of the T-iPS cells can be performed by, for example, detecting the state of TCR gene rearrangement by genomic PCR.

The CD8 single-positive cells thus obtained can be isolated by appropriately selecting a known method. Examples of the known method include flow cytometry using an antibody against a cell surface marker of CD8 and a cell sorter. For example, in the case of CD8 single-positive cells, a method for purifying the CD8 single-positive cells using, for example, an affinity column on which an antigen recognized by T cells that are the origin of the CD8 single-positive cells is immobilized, or a method for purifying the CD8 single-positive cells using an MHC multimer (for example, MHC tetramer) to which the antigen is bound can also be adopted.

The CD8 single-positive cells obtained by the present invention do not express PD-1, but express CCR7 together with CD27 and CD28 representing phenotypes of central memory T cells, and also have telomeres longer than those of the original T cells, and have high self-replication ability. Therefore, according to the present invention, it is possible to produce CD8 single-positive T cells having the same rearrangement pattern of a TCR gene as that of the original T cells, the CD8 single-positive T cells not expressing PD-1 but expressing CD27, CD28, and CCR7. T cells collected from humans are different from the obtained T cells in that the T cells collected from humans express PD-1 and the proportion of immature memory phenotypes is small.

In order to maintain the CD8 single-positive cells thus obtained, the cells may be stimulated every 1 to 2 weeks. Examples of such stimulation include contact with at least one substance selected from the group consisting of an anti-CD3 antibody, an anti-CD28 antibody, IL-2, IL-7, IL-15, an antigen recognized by the CD8SP cell, an MHC multimer to which the antigen is bound, feeder cells having an allogeneic relationship with the CD8 single-positive cells, and feeder cells having an autologous relationship with the CD8 single-positive cells.

The thus obtained cytotoxic T cells derived from human T cell-derived iPS cells expressing HLA class I of HLA-A24 and HLA-E of the present invention are useful as cytotoxic T cells derived from human T cell-derived iPS cells that can avoid an NK cell missing-self response and that can be used for allogeneic administration while maintaining the antigen-specific cytotoxicity of human T cells used as raw materials. The NK cell missing-self responsiveness of the cytotoxic T cells of the present invention is significantly lower than that of T cells expressing only HLA-A24 or HLA-E.

### Examples

Next, the present invention will be described in more detail with reference to Examples, but the present invention is not limited to these Examples.

### Example 1

Establishment of T-iPS cells from human papillomavirus (HPV)-specific CTL clones using a Sendai virus vector.
1) Peripheral blood mononuclear cells were separated from the peripheral blood of a healthy person, and then dendritic cells were induced for the purpose of antigen presentation. After 7 days, an HPV antigen peptide (HPV16-E6, A2402) was added to the induced dendritic cells, and co-culture with peripheral blood mononuclear cells was initiated. After about 8 to 10 days, for HPV-specific CTL detection, CTLs were stained with an MHC tetramer, and then the tetramer-positive rate was confirmed by flow cytometry. After HPV-specific CTLs were confirmed, a limiting dilution method after single-cell sorting or tetramer/PE bead selection was performed, and PBMC after 50 Gy X-ray irradiation, IL2, and PHA were added for stimulation.
2) Tetramer staining of colonies raised after about 3 to 6 weeks was performed, and establishment of CTL clones was confirmed by flow cytometry. After confirmation of establishment, the CTL clones were stimulated with CD3/28, and then subjected to gene introduction using the following two vectors A). The CTLs after gene introduction were transferred to a 6-well plate coated with iMatrix, and culture was initiated in a CO₂ incubator using a CTL medium as a medium.

### A) SeV4 factor vector + SV40 large T antigen

3) On the next day of SeV gene introduction, an equal amount of an iPS medium (StemFitAK03N) was added, and thereafter, the iPS medium was replaced with Stem FitAK03N by half every other day.
4) Colonies of T-iPS cells were observed after 7 days, and then colony pick-up was performed.

### Example 2

Knockout of all HLA class I of HPV antigen-specific CTL-derived iPS cells.

First, β2 microglobulin (B2M) is knocked out. Five micrograms each of a knockout plasmid and a guide RNA were electroporated using LONZA 4-D Nucleofector into T-iPSCs after cell detachment. Thereafter, the cells are seeded in 3 wells of a 6-well plate and cultured. Since the knockout plasmid contained a target sequence of the guide RNA used for the second editing, GFP, and a selection marker of CD8, MACS bead positive selection of CD8 was performed at a timing when the cells seeded in 3 wells became confluent after about 10 days. After selection, iPS cells were single-cell cloned by thinly seeding the T-iPSCs. GFP strongly positive cells were picked up and then cultured, genotyping was performed, and a clone having a marker biallelically was identified by PCR and then expanded, and the process proceeded to the next step.

### Example 3

### Introduction of genes of HLA-A24 and HLA-E.

Since the epitope was A2402-restricted, HLA-A2402 was knocked in to T-iPSCs after B2M knockout. At the same time, a knock-in plasmid having an HLA-E trimer structure was also prepared, and HLA-E was knocked in. Furthermore, a plasmid in which half of HLA-A2402 and half of HLA-E were knocked in at the same time was also prepared. After cell detachment of T-iPSCs in which B2M was knocked out in the first editing, electroporation was performed using LONZA 4-D Nucleofector. After electroporation, the cells were seeded in 3 wells of a 6-well plate and cultured. After about 7 days, negative selection of CD8 was performed using MACS beads at a timing when the cells seeded in 3 wells became confluent. After selection, iPS cells were single-cell cloned by thinly seeding the T-iPSCs. GFP negative cells were picked up and then cultured, genotyping was performed, and clones were identified and then expanded.

### Example 4

Redifferentiation of T-iPS cells after genome editing into CD8 single-positive T cells.

A small mass of the T-iPS cells after genome editing obtained in Example 3 was transferred onto C3H10T1/2 cells, and co-cultured in an EB medium in the presence of 20 ng/mL of VEGF, 50 ng/mL of SCF, and 50 ng/mL of FLT-3L. On Day 14 of culture, hematopoietic cells contained in the iPS sack were collected, and the cells were transferred onto cells on DL1/4-expressing C3H10T1/2 cells, and the hematopoietic cells were differentiated into T lineage cells in an OP9 medium in the presence of 10 ng/mL of FLT-3L and 1 ng/mL of IL-7.

Then, on Day 42 of culture, the cells were stimulated by adding α-CD3/CD28 beads or 5 µg/ml of PHA to the OP9 medium.

Then, the T lineage cells were collected and cultured in a CTL medium together with irradiated PMBCs in the presence of 10 ng/mL of IL-7 and 10 ng/mL of IL-15.

Then, on Day 56 of culture, CD8/tetramer-positive cells were observed. HLA of the cells was examined by FACS analysis, and knocked-in HLA genes (HLA-A24, HLA-E, or both) were expressed.

### Example 5

Antigen specificity of CD8 single-positive cells of the present invention.

It was examined whether the redifferentiated cells obtained in Example 4 had the same antigen specificity as that of the original T cells even after genome editing. As a result, it was revealed that the antigen specificity of the obtained redifferentiated CD8 single-positive cells was consistent with the antigen specificity of the original HPV-T cell clones (Fig. 1).

After successful induction of differentiation of rejuvenated CTLs (rejT) expressing only HLA-A24, or only HLA-E, or both, a chromium assay and a CD107a assay were performed to examine if the rejT could avoid an NL cell missing-self response. Although HPV-rejT expressing only HLA-A24 and only HLA-E suppressed NK activity, the suppression was insufficient, while HPV-rejT expressing both could significantly suppress the cytotoxic activity of NK cells. It was demonstrated that it is important to express both an HLA-restricted HLA class I molecule of an antigen epitope of a CTL and HLA-E in order to suppress NK activity (Figs. 2 and 3).

### Example 6

Effect on prolonging the survival time of HLA-edited HPV-rejT in cervical cancer-bearing mice.

### (Method)

Immunodeficient mice (NOG mice) were intraperitoneally transplanted with a cervical cancer cell line SiHa, and then divided into an untreated control group and three treatment groups (original HPV-CTL clone, wild type (WT) HPV-rejT, or HLA-edited HPV-rejT), and 2.5 × 10⁶ T cells were administered once a week and three times by intraperitoneal injection. The survival times were compared for the purpose of confirming the therapeutic effects on the mice in the untreated control group and the respective treatment groups.

### (Results)

The survival time of the cervical cancer-bearing mice is shown in Fig. 4.

As shown in Fig. 4, the survival time of the cervical cancer-bearing mice was significantly prolonged in the WT rejT administration group and the HLA-edited HPV-rejT (EXrejT) administration group compared with the original CTL administration group.

In pathological findings of the treated mice that survived for 6 months or more, no tumor remained in the lung, liver, intestinal tract, spleen, and uterus.

### Example 7

Durability in vivo of HLA-edited HPV-rejT upon co-administration of NK cells.

### (Method)

After intraperitoneal transplantation of a cervical cancer cell line SiHa into immunodeficient mice (NOG mice) on Day 4, the mice were divided into three groups.
1. HLA-edited FFluc-rejT + NK cells (HLA-A24+)
2. HLA-edited FFluc-rejT + NK cells (HLA-A24-)
3. HLA-edited FFluc-rejT

On Day 0, groups 1 and 2 received 2.5 × 10⁶ rejT cells + 2.5 × 10⁶ NK cells and group 3 received 2.5 × 10⁶ rejT cells via intraperitoneal injection. Proliferation and persistence of rejT in vivo of each group were monitored by IVIS and compared.

### (Results)

The results are shown in Fig. 5. In group 3 to which NK cells were not administered, fluorescently labeled HPV-rejT efficiently proliferated and persisted in vivo on Day 7, whereas in group 2, fluorescently labeled HPV-rejT was eliminated by NK cells not having HLA-A24, and rejT could be detected on Day 7. Meanwhile, in group 1, it was confirmed that fluorescently labeled HPV-rejT was not eliminated by NK cells having HLA-A24, and efficiently proliferated and persisted in vivo (left figure). When persistence of fluorescently labeled HPV-rejT on Day 7 in mice in vivo was confirmed by a signal, the signal in group 1 was significantly higher than that in group 2. On the other hand, there was no significant difference from group 3 (right figure).

## Claims

1. A cytotoxic T cell derived from a human T cell-derived iPS cell expressing HLA class I of HLA-restricted class I of an antigen epitope of a CTL and HLA-E.

2. The cytotoxic T cell derived from a human T cell-derived iPS cell according to claim 1, wherein the HLA-restricted class I molecule of the antigen epitope of the CTL is HLA-A24 or HLA-A02.

3. A method for producing a cytotoxic T cell derived from a human T cell-derived iPS cell expressing two types of HLA class I of HLA-restricted class I of an antigen epitope of a CTL and HLA-E, the method comprising: a step of knocking out all HLA class I of the human T cell-derived iPS cell; a step of introducing a gene of the HLA-restricted HLA class I of the antigen epitope of the CTL and a gene of the HLA-E into the T-iPS cell in which all HLA class I have been knocked out; and a step of redifferentiating the genetically introduced T-iPS cell into a CD8 single-positive T cell.

4. The production method according to claim 3, wherein the HLA-restricted class I molecule of the antigen epitope of the CTL is HLA-A24 or HLA-A02.
